# EUROPÄISCHE PATENTSCHRIFT

(11) **EP 3 694 516 B1**
(45) Veröffentlichungstag und Bekanntmachung des Hinweises auf die Patenterteilung: **13.12.2023**
(21) Anmeldenummer: 18786271.9
(22) Anmeldetag: 10.10.2018
(51) Int. Cl.: A61K 31/4709, A61P 31/04, C07D 401/12

(54) **BIOLOGISCH ABBAUBARE CHINOLON-ANTIBIOTIKA**
BIODEGRADABLE QUINOLONE ANTIBIOTICS
ANTIBIOTIQUES BIODÉGRADABLES DE LA FAMILLE DES QUINOLONES

(30) Priorität: 11.10.2017 DE 102017218114
(43) Veröffentlichungstag der Anmeldung: 19.08.2020
(73) Patentinhaber: Leuphana Universität Lüneburg Stiftung Öffentlichen Rechts, 21335 Lüneburg (DE)
(72) Erfinder: KUEMMERER, Klaus, 21337 Lueneburg (DE); LEDER, Christoph, 21403 Wendisch Evern (DE); RASTOGI, Tushar, 21335 Lueneburg (DE); SUK, Morten, 21337 Lueneburg (DE); MENZ, Jakob, 21339 Lueneburg (DE)
(74) Vertreter: Wichmann, Hendrik
(86) Internationale Anmeldenummer: PCT/EP2018/077584
(87) Internationale Veröffentlichungsnummer: WO 2019/072907

(56) Entgegenhaltungen:
- US-A- 4 382 089
- US-A- 4 416 884
- US-B2- 8 889 689
- KLAUS KÜMMERER: "Sustainable from the very beginning: rational design of molecules by life cycle engineering as an important approach for green pharmacy and green chemistry", GREEN CHEMISTRY, Bd. 9, Nr. 8, 1. Januar 2007 (2007-01-01), Seite 899, XP055524225, GB ISSN: 1463-9262, DOI: 10.1039/b618298b
- QING-RONG QI ET AL: "Synthesis and antibacterial activity of new fluoroquinolones containing a cis- or trans-cyclohexane moiety", BIOORGANIC & MEDICINAL CHEMISTRY LETTERS, Bd. 22, Nr. 24, 1. Dezember 2012 (2012-12-01), Seiten 7688-7692, XP055522716, AMSTERDAM, NL ISSN: 0960-894X, DOI: 10.1016/j.bmcl.2012.09.106
- H-G WETZSTEIN ET AL: "Patterns of metabolites produced from the fluoroquinolone enrofloxacin by basidiomycetes indigenous to agricultural sites", APPLIED MICROBIOLOGY AND BIOTECHNOLOGY, SPRINGER, BERLIN, DE, Bd. 71, Nr. 1, 3. Dezember 2005 (2005-12-03), Seiten 90-100, XP019421924, ISSN: 1432-0614, DOI: 10.1007/S00253-005-0178-4
- GUO HONGGUANG ET AL: "Kinetics and transformation pathways on oxidation of fluoroquinolones with thermally activated persulfate", CHEMICAL ENGINEERING JOURNAL,, Bd. 292, 11. Januar 2016 (2016-01-11), Seiten 82-91, XP029448671, ISSN: 1385-8947, DOI: 10.1016/J.CEJ.2016.01.009
- SAUTER F ET AL: "Synthesis of novel quinolone-type drugs. Part 4. Pyrido[3,2,1-gh][1,7]phenanthroline- and benzo[i,j]quinolizinecarboxylic acids", SCIENTIA PHARMACEUTICA, OESTERREICHISCHE APOTHEKER-VERLAGSGESELLSCHAFT MBH, AUSTRIA, Bd. 57, Nr. 1, 1. Januar 1989 (1989-01-01) , Seiten 7-20, XP009509318, ISSN: 0036-8709
- Mark P. Wentland ET AL: "Chapter 15. Quinolone Antibacterial Agents" In: "ANNUAL REPORTS IN MEDICINAL CHEMISTRY", 1. Januar 1985 (1985-01-01), Academic Press, US, XP055314055, ISSN: 0065-7743 Bd. 20, Seiten 145-154, DOI: 10.1016/S0065-7743(08)61041-6, Verbindungen 4, 5, 7-9 , 11
- DATABASE CAPLUS [Online] CHEMICAL ABSTRACTS SERVICE, COLUMBUS, OHIO, US; 28 August 2016 (2016-08-28), Halehatty S.Bhojya Naik ET AL: "ANTIMICROBIAL COMPOUNDS, SYNTHESIS AND APPLICATIONS THEREOF, IN 2015CH00032", XP055916389, retrieved from STN Database accession no. 216:1429194 -& Halehatty Naik S. Bhojya: "ANTIMICROBIAL COMPOUNDS, SYNTHESIS AND APPLICATIONS THEREOF"", PATENT APPLICATION, 1 January 2016 (2016-01-01), XP055916313, Retrieved from the Internet: URL:https://content2.cas.org/v1/AUTH_9a355 bb5cefd4c378bde0d541c6a11ce/patentpak-cdr- full-text-18/patent/76871612_1537160410.pd f?temp_url_sig=f5388647d1e6911573a04f8e60c bd36d1d5e45ee&temp_url_expires=1651187138& inline

## Beschreibung

Die vorliegende Erfindung betrifft neue Chinolon-Antibiotika zur medizinischen Verwendung, wie in den Ansprüchen definiert.

### Stand der Technik

Da sich die instrumentelle Analytik in den letzten Jahrzehnten rasant weiterentwickelt hat, wurden bisher schon mehr als hundert verschiedene Pharmazeutika im Ablauf von Kläranlagen, Oberflächengewässern, Grundwasser und vereinzelt auch Trinkwasser im Bereich von ng/L bis µg/L detektiert. Diese Mikroverunreinigungen werden daher mittlerweile als Risiko für die Wasserqualität und ein nachhaltiges Management der Wasserressourcen wahrgenommen. In dem Zusammenhang ist auch sehr wenig über das Auftreten und das umweltwissenschaftliche Schicksal der Zerfalls- und Abbauprodukte der applizierten oder unsachgemäß entsorgten Pharmazeutika bekannt. Persistenz, Akkumulation, Metabolisierung durch Mikroorganismen, Tiere und Pflanzen, Reaktion mit Sauerstoff oder Beeinflussung durch Licht können die Ausgangsstoffe so verändern, dass sich die Toxizität der Substanzen verändern kann und damit die Trinkwasserresourcen beeinträchtigt werden können.

Besonders problematisch sind Antibiotika. Antibiotika werden zur Behandlung von Infektionen mit pathogenen Bakterien genutzt. Übermäßige Nutzung von nicht abbaubaren Antibiotika durch menschliche Patienten oder bei Nutztieren könnte zur Akkumulation im Wasser führen und anschließend die Resistenzentwicklung fördern, wenn große Areale mit relevanten Bakterien ausreichende Wirkstoffkonzentrationen von Antibiotika haben und somit ständigen Selektionsdruck auf die Bakterien ausüben.

Es ist bekannt, dass biologisch abbaubare Verbindungen chemische Bindungen enthalten können, die beispielsweise enzymatisch spaltbar sind. Exemplarisch seien Esterbindungen, Amidbindungen oder Acetalbindungen genannt.

CN103405435A und CN102030737A offenbaren Aminoderivate (enthalten eine Amidbindung in einer Seitenkette, jedoch nicht am Grundgerüst des Antibiotikums) des Antibiotikums Clinafloxacin).

Weiterhin offenbart die EP1160241A2 die Synthese von antimikrobiell wirkenden Fluorchinolon-Derivaten, wobei jedoch Amidbindungen nicht explizit erwähnt werden.

EP373531A1 offenbart ebenfalls antibiotisch wirkende Fluorchinolon-Grundstrukturen (ausgehend von Ofloxacin), die jedoch keine Amidbindungen enthalten.

*Wetzstein et al. 2006* betrifft Enrofloxacin-Metabolite, die durch Behandlung von Pilzen mit Enrofloxacin erzeugt worden sein sollen. Das Dokument schlägt viele Strukturen von Metaboliten vor, die allerdings, wenn überhaupt, nur aufgrund der Massenspektren über LC-MS der Reaktionsmischungen vermutet worden sind (nur hochauflösende Massenspektrometrie ohne spezifische Isolierung und keine Strukturbestätigung nach Synthese und nachfolgendem NMR). Die vorliegend beanspruchten Antibiotika werden somit nicht beschrieben.

Das Chinolon-Antibiotikum der vorliegenden Erfindung ist keine der in *Wetzstein et al.* 2006skizzierten Verbindungen, wie die Verbindungen auf Seite 94 von *Wetzstein et al. 2006,* insbesondere nicht Verbindung 106 von *Wetzstein et al. 2006*: 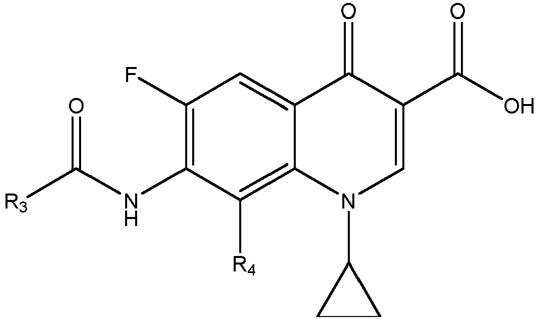 worin R₃ für -CH₂-NH₂ steht. H

WO 2007/106537 A2, JP 57016882 A und *Sauter et al. 1989* offenbaren Antibiotika, aber keine der vorliegend beanspruchten Verbindungen.

Keine der hierin erwähnten Veröffentlichungen offenbart das der vorliegenden Erfindung zugrundeliegende Konzept. Die hierein beanspruchten Verbindungen sollen eine ausreichende Stabilität aufweisen, um für einen ausreichenden Zeitraum im menschlichen oder tierischen Körper wirken zu können (z.B. für 1-3 Tage), wohingegen die Verbindungen nach dem Ausscheiden aus dem Körper relativ schnell (d.h. innerhalb weniger Wochen oder Monaten), beispielsweise unter Einfluss von UV-Licht, abgebaut werden und dann nicht mehr antibiotisch wirken oder zumindest weniger antibiotische Aktivität aufweisen. Dies wird durch die erfindungsgemäße Amid-Bindung am Grundgerüst des Antibiotikums sichergestellt.

Die Publikation von Junza et al. offenbart, dass Fluorchinolon-Antibiotika (z.B. Ciprofloxacin) und deren Abbauprodukte in Kuhmilchproben nachgewiesen wurden. Hier wurden auch Ciprofloxacin-Aminoderivate nachgewiesen, welche Amidbindungen in einer Seitenkette, jedoch nicht am Grundgerüst des Antibiotikums enthielten (Journal of Agricultural and Food Chemistry, 62(8), 2008-2021).

Desweiteren zeigen Liu et al., dass Fluorchinolon-Antibiotika (z.B. Ciprofloxacin) und deren Abbauprodukte in durch Ozonisierung aufgereinigtem Abwasser nachgewiesen werden konnten. Hier wurden ebenfalls Abbauprodukte (Aminoderivate) nachgewiesen, welche Amidbindungen in einer Seitenkette, jedoch nicht am Grundgerüst des Antibiotikums enthielten (Water Research 2012, 46(16), 5235-5246).

Weitere Chinolon-Antibiotika sind in der folgenden Literatur beschrieben:
- Klaus Kümmerer: "Sustainable from the very beginning: rational design of molecules by life cycle engineering as an important approach for green pharmacy and green chemistry", GREEN CHEMISTRY, Bd. 9, Nr. 8, 2007, Seiten 899-907
- Qing-Rong Qi ET AL: "Synthesis and antibacterial activity of new fluoroquinolones containing a cis- or trans-cyclohexane moiety", Bioorganic & Medicinal Chemistry Letters, Bd. 22, Nr. 24, 2012, Seiten 7688-7692
- US 8 889 689
- GUO HONGGUANG ET AL: "Kinetics and transformation pathways on oxidation of fluoroquinolones with thermally activated persulfate", CHEMICAL ENGINEERING JOURNAL, Bd. 292, 1 1 . 201 6, Seiten 82-91
- SAUTER F ET AL: "Synthesis of novel quinolone-type drugs. Part 4. Pyrido[3,2,1-gh][1,7]phenanthroline- and benzo[i,j]quinolizinecarboxylic acids", SCIENTIA PHARMACEUTICA, Bd. 57, Nr. 1, 1989, Seiten 7-20
- US 4 416 884
- Mark P. Wentland ET AL: "Chapter 15. Quinolone Antibacterial Agents" In: "ANNUAL REPORTS IN MEDICINAL CHEMISTRY", 1985, Bd. 20, Seiten 145-154
- DATABASE CAPLUS [Online] CHEMICAL ABSTRACTS SERVICE, COLUMBUS, OHIO, US; 28. August 201 6, Halehatty S.Bhojya Naik ET AL: "ANTIMICROBIAL COMPOUNDS, SYNTHESIS AND APPLICATIONS THEREOF, IN 2015CH00032", XP055916389, STN Database accession no. 216:1429194; & PATENT APPLICATION, "ANTIMICROBIAL COMPOUNDS, SYNTHESIS AND APPLICATIONS THEREOF" 2016, XP055916313,

### Zusammenfassung der Erfindung

Im Rahmen der vorliegenden Erfindung wurden umweltfreundliche (Schmalband)-Antibiotika-Varianten auf Basis der Fluorchinolon-Grundstruktur entwickelt, um nachfolgend die durch Pharmazeutika verursachten Gefahren für die Wasserqualität abzumildern, indem die biologische Abbaubarkeit verbessert wird oder zumindest die Toxizität von nicht-abbaubaren Metaboliten, Transformationsprodukten und Fragmenten neutralisiert wird.

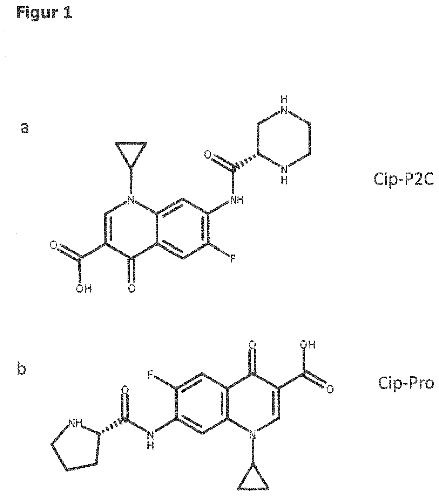

Mehrere Derivate von Ciprofloxacin (siehe Figur 1) wurden synthetisiert und in verschiedenen Bakterien getestet. Die Derivate leiten sich durch Verknüpfung der Grundstruktur mit Biomolekülen über hydrolysierbare Bindungen (hier Amid-Bindungen) ab. Diese Hydrolyse kann biologisch (enzymatisch) erfolgen.

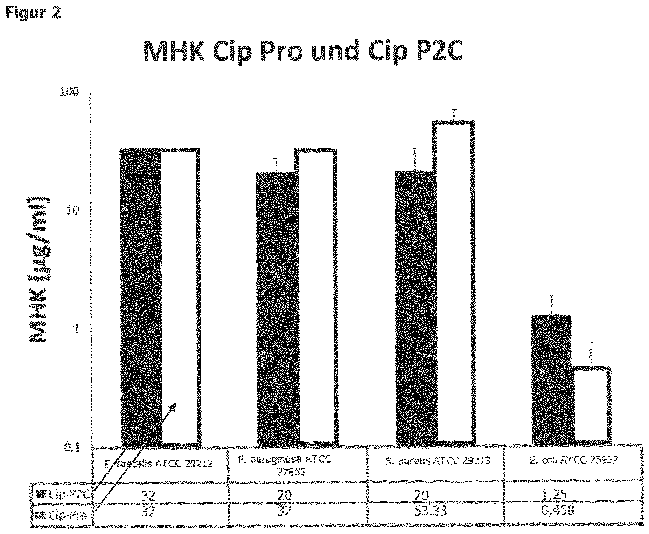

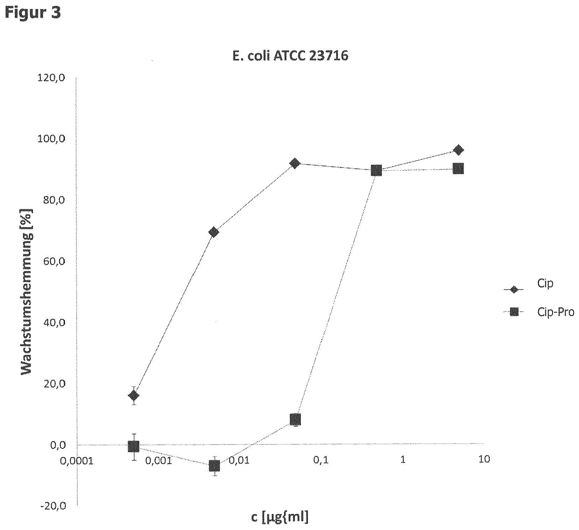

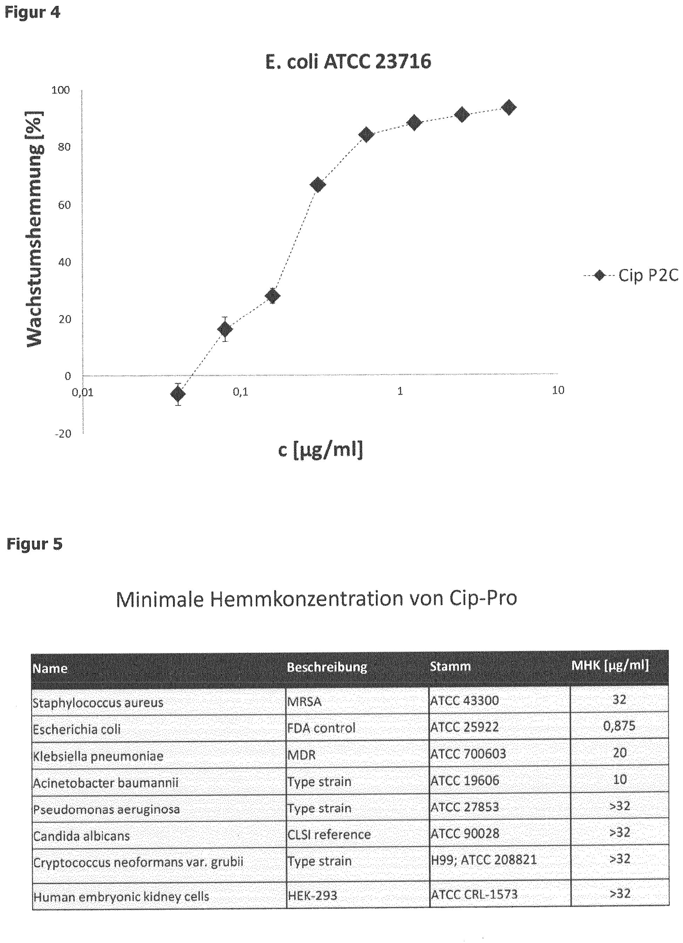

Es zeigte sich, dass die zwei Derivate (siehe Figur 1) besonders aktiv in E. coli waren (siehe Figur 2). Die minimalen Hemmkonzentrationen lagen hier in der Größenordnung verschiedener etablierter Fluorchinolone. Dies wurde durch eigene Daten in Wachstumstests in E.coli (siehe Figur 3, 4 und 5) bestätigt. In weiteren Bakterien waren sie jedoch deutlich schwächer aktiv (siehe Figur 2 und 5), so dass eine spezifische Behandlung von E. coli Infektionen (z.B. Harnwegsinfektionen) ohne kollateralen Selektionsdruck auf andere Keime möglich wäre. Das Ergebnis wäre unter Umständen eine mildere Behandlung dieser Erkrankungen mit weniger Risiko einer ausgeprägten Resistenzbildung in anderen Bakterien.

Dadurch können neue Medikamente entstehen, die pharmakologisch und umweltwissenschaftlich verbessert sind.

Obwohl Amidasen im Menschen vorkommen und daher theoretisch zu einer Inaktivierung des neuen Antibiotikums führen würden, so wurde unerwartet festgestellt, dass die beiden Derivate keine erhöhte Instabilität durch Leberenzyme und intestinale Enzyme und eine hinreichende abiotische Stabilität unter physiologischen Bedingungen aufzuweisen scheinen. Die langfristige Abbaubarkeit, beispielsweise durch Mikroorganismen, im Ökosystem sollte somit nicht im Gegensatz zum Einsatz als Medikament stehen.

Insbesondere sollte die erfindungsgemäße Amid-Bindung in der Zirkulation im menschlichen Körper ähnlich stabil sein wie hydrolysierbare Amid-Bindungen bei anderen biotechnologischen Medikamenten (Antikörper, Proteine, Peptide).

Nicht auszuschließen ist, dass sich auch gegen diese neuen Antibiotika-Varianten Resistenzen bilden könnten. Es besteht aber die Möglichkeit, dass dies deutlich weniger wahrscheinlich ist als mit den Breitband-Fluorchinolonen. Das heißt, nur Bakterienstämme, die durch das Medikament ausreichend unter Selektionsdruck gesetzt werden, können gegen Fluorchinolone resistent werden. Die hierin beschriebenen Derivate könnten eine spezifische/selektive Wirkung auf *E. coli* Bakterien, nicht aber auf andere pathogene Keimen haben, so dass erreicht würde, dass bei Behandlung diagnostizierter *E. coli* Infektionen lediglich die bekämpften Pathogene resistent werden könnten. Das Risiko einer Resistenzbildung wäre also minimiert. Bisher werden Fluorchinolone von den Fachgesellschaften nicht als erste Wahl bei Harnwegsinfektionen (ca. 70% E. coli Infektionen) empfohlen, um die Breitbandwirkung nicht durch Resistenzen zu verlieren.

Durch die gute Abbaubarkeit der während der Therapie vom Menschen/Tier ausgeschiedenen Antibiotika wird der auf Umweltkeime einwirkende Selektionsdruck und die Resistenzausbildung eingedämmt.

### Beschreibung der Figuren

**Figur 1****:** Molekulare Strukturen der beiden Fluorchinolon-Derivate Cip-P2C (a) und Cip-Pro (b).
**Figur 2****:** Antibiotische Aktivität von Cip-Pro und Cip-P2C (I). Minimale Hemmkonzentrationen [µg/ml] von Cip-Pro und Cip-P2C in verschiedenen Bakterien (E. faecalis ATCC 29121, P. aeruginosa ATCC 27853, S. aureus ATCC 29213, E. coli ATCC 25922).
**Figur 3****:** Wachstumshemmung von E. coli durch Cip-Pro im Vergleich zu Ciprofloxacin. Dosis-Wirkungsbeziehung der Wachstumshemmung von E. coli ATCC 23716 durch Cip-Pro im Vergleich zu Ciprofloxacin.
**Figur 4****:** Wachstumshemmung von E. coli durch Cip-P2C. Dosis-Wirkungsbeziehung der Wachstumshemmung von E. coli ATCC 23716 durch Cip-P2C.
**Figur 5****:** Antibiotische Aktivität von Cip-Pro (II). Minimale Hemmkonzentrationen [µg/ml] in verschiedenen Bakterienstämmen und Zytotoxizität in humanen HEK 293 Zellen von Cip-Pro in verschiedenen Bakterien (S. aureus ATCC 43300, E. coli ATCC 25922, K. pneumoniae ATCC 700603, A. baumanii ATCC 19606, P.aeruginosa ATCC 27853 und humane embryonale Nierenzellen (HEK 293 CRL-1573).

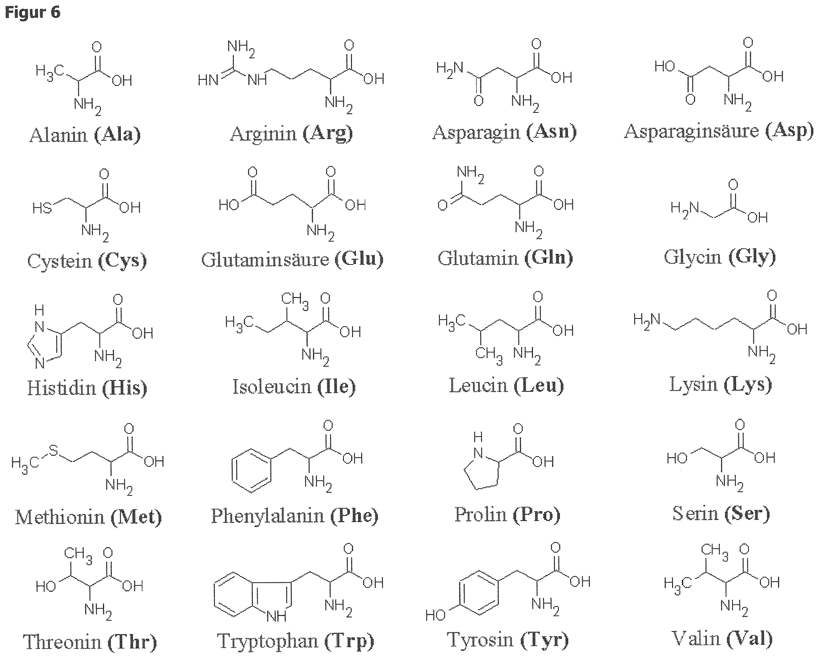

**Figur 6****:** Natürliche Aminosäuren.
**Figuren 7a****-c:** Abiotische Hydrolyse von Ciprofloxacin, Cip-Pro, Cip-P2C. Im Rahmen der Erfindung kann ausgenutzt werden, dass der pH-Wert unter physiologischen Bedingungen anders ist als z.B. im kommunalen Abwasser (meist pH 8-9), und dass jeweils andere Bakterien vorhanden sind, so dass die Anforderungen an die physiologische Stabilität und die gewünschte Instabilität im Abwasser unterschiedlich sind.
**Figur 8****:** Enzymatische Hydrolyse durch intestinale Mikrosomen von Ciprofloxacin, Cip-Pro, Cip-P2C.
**Figur 9****:** Enzymatische Hydrolyse durch humane intestinale Mikrosomen (Xenotech) oder 500 µg/ml humane Lebermikrosomen (Corning).
**Figur 10****:** Beschreibung eines möglichen Syntheseschemas für CIP-P2C.

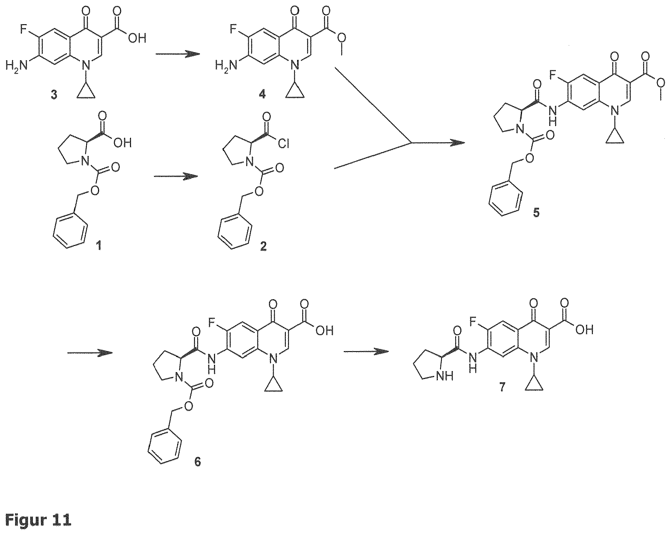

**Figur 11****:** Beschreibung eines möglichen Syntheseschemas für CIP-Prolin.

### Detaillierte Beschreibung der Erfindung

Die vorliegende Erfindung betrifft die folgenden Ausführungsformen:
1. Chinolon-Antibiotikum der allgemeinen Formel I, II, III oder IV: 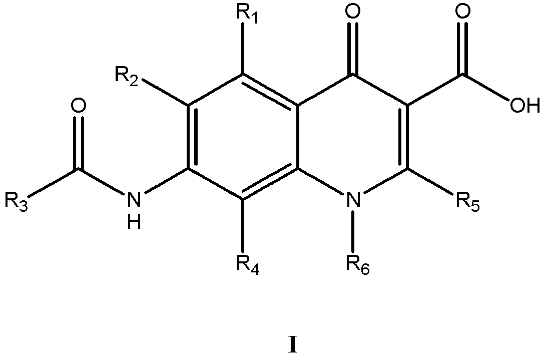 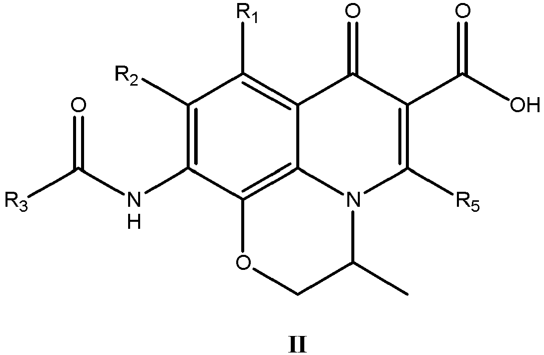 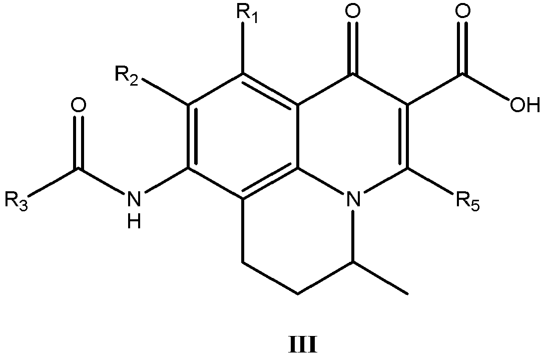 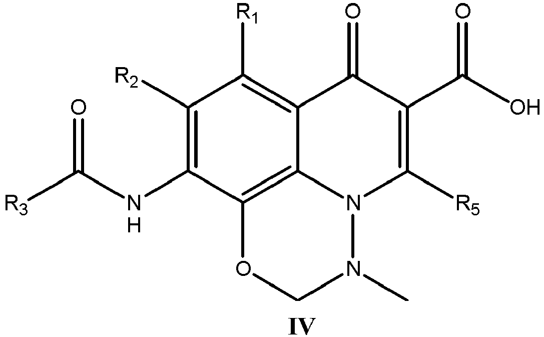 worin
R₁ bis R₅ die in Anspruch 1 genannte Bedeutung haben
und R₆ ausgewählt ist aus

| Cyclopropyl | Cyclobutyl | Ethyl | Propyl | Methyl |
|---|---|---|---|---|
| 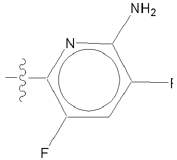 | 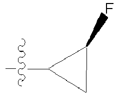 | 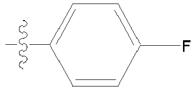 | 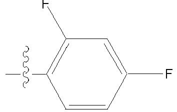 | 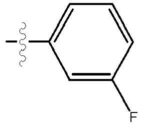 |
| 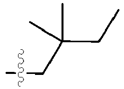 | 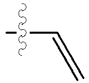 | 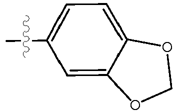 | 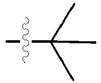 | 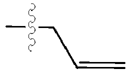 |
| 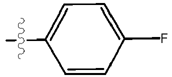 | 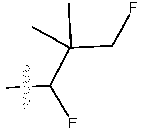 | 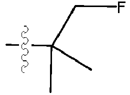 | 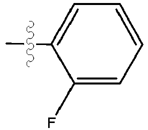 | 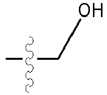 |
| 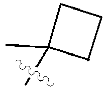 | 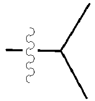 | 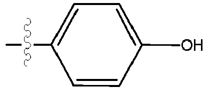 | 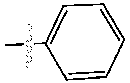 | 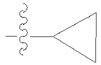 |

Bevorzugte Kombinationen für R1 bis R5 sind:

| Strukturnummer | R1 | R2 | R3 | R4 | R5 |
|---|---|---|---|---|---|
| 1 | -H | -F | Wie in den Ansprüchen hierin definiert | -H | -H |
| 2 | -H | -H | Siehe Strukturnr. 1 | -OCH₃ | -H |
| 3 | -H | -H | Siehe Strukturnr. 1 | -OCHF₂ | -H |
| 4 | -H | -F | Siehe Strukturnr. 1 | -Cl | -H |
| 5 | -H | -F | Siehe Strukturnr. 1 | -CN | -H |
| 6 | -H | -F | Siehe Strukturnr. 1 | -OCHF₂ | -H |
| 7 | -H | -F | Siehe Strukturnr. 1 | -OCH₃ | -H |
| 8 | -H | -F | Siehe Strukturnr. 1 | -F | -H |
| 9 | -F | -F | Siehe Strukturnr. 1 | -F | -H |
| 10 | -NH₂ | -F | Siehe Strukturnr. 1 | -F | -H |

Die erfindungsgemäßen Chinolon-Antibiotika könnten beispielsweise durch Amidasen abgebaut werden. Zudem ist bekannt, dass Chinolon-Antibiotika mit den oben angegebenen Resten R₁, R₂ und R₅ antibiotisch wirksam sind. Wie vorliegend gezeigt, führt das Einführen einer abspaltbaren Gruppe an R₃ nicht dazu, dass diese antibiotische Wirksamkeit verloren geht.

Soweit nicht anders angegeben, zielt die vorliegenden Erfindung auf alle Stereoisomere ab. Da natürliche Aminosäuren hauptsächlich in der L-Form vorkommen, wird davon ausgegangen, dass die L-(Stereo-)Isomere des Rests R3 verstärkt enzymatisch abgespalten werden können. Der Abbau kann sowohl abiotisch als auch biotisch (enzymatisch) erfolgen.

Der Ausdruck "der Rest einer natürliche Aminosäure nach amidischer Bindung an das Chinolon-Grundgerüst", so wie hier verwendet, bedeutet, dass die Aminosäure amidisch an das Chinolon-Gerüst gekoppelt wird und dann natürlich nicht mehr als "Säure" vorliegt, sondern unter Wasserabspaltung Teil der Amidbindung wird. Die zu R₃ benachbarte Carbonylgruppe stammt aus der Säurefunktion der Aminosäure,.

Das Chinolon-Antibiotikum kann beispielsweise folgende Formel aufweisen: 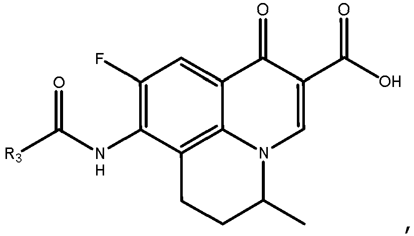 oder 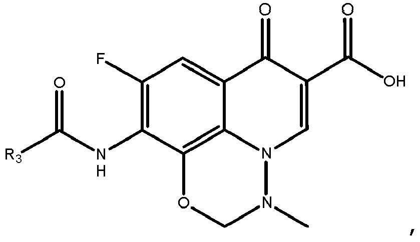 oder

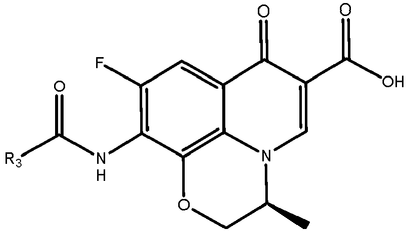

Das Chinolon-Antibiotikum kann beispielsweise folgende Reste aufweisen:
R₁ = H, oder F (dies gilt für Formel I und Formel IV)
R₂ = H, oder F
R₃ = wie in Ausführungsform 1 definiert
R₄ = H, OCH₃, OCHF₂, Cl, CN, oder F, und
R₅ = H.

Das Chinolon-Antibiotikum kann beispielsweise die Formel II-a und II-b aufweisen (wobei Formel IIb nicht anspruchsgemäß ist)

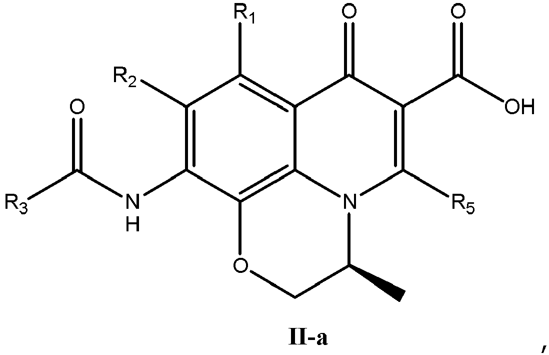

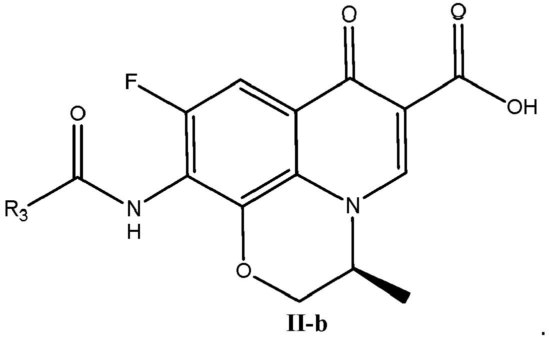

Das Chinolon-Antibiotikum kann die allgemeine Formel I-a aufweisen:

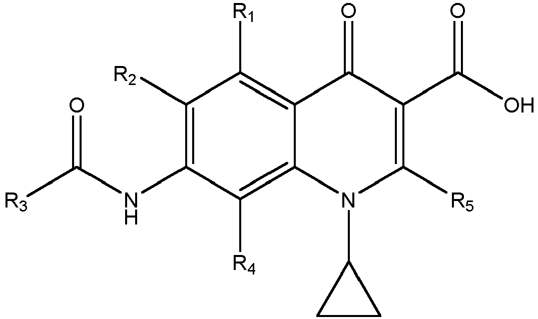

Das Chinolon-Antibiotikum kann beispielsweise die folgende Formel aufweisen Reinschrift 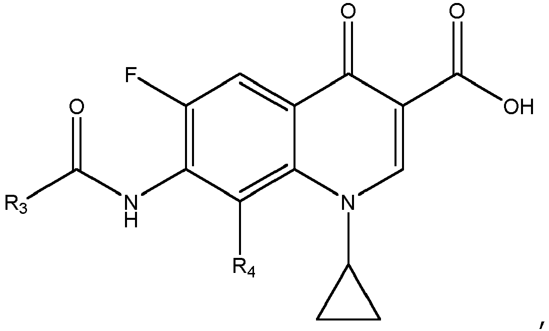 wobei
R₃ = der Rest einer Aminosäure wie in Anspruch 1 definiert, insbesondere L-Prolin, und wobei R₄ wie oben definiert ist.

Das Chinolon-Antibiotikum kann eine der folgenden Formeln aufweisen:

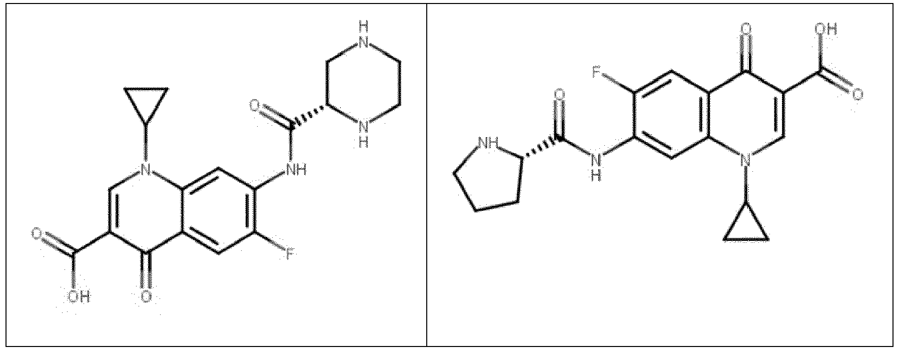

Die Amidbindung kann abiotisch oder biotisch abgespalten werden.

Die Erindung betrifft auch ein Medikament enthaltend ein erfindungsgemäßes Chinolon-Antibiotikum.

Die hierin beschriebenen Antibiotika können auf übliche weisen formuliert bzw. in Medikamente/Dosierungsformen eingebracht werden.

Das erfindungsgemäße Chinolon-Antibiotikums kann als Medikament verwendet werden.

Das erfindungsgemäße Chinolon-Antibiotikum kann zur Behandlung von bakteriellen Infektionen verwendet werden.

Insbesondere betrifft die Erfindung die hierin beschriebenen Antibiotika zur Verwendung gegen Krankenhauskeime (nosokomiale Keime), bzw. Keime, die behandelt werden sollen ohne eine Resistenzbildung bei anderen Keimen zu riskieren. Insbesondere betrifft die Erfindung die hierin beschriebenen Antibiotika zur Behandlung von *E*. *Coli*-verursachten Infektionen.

### Beispiele

### 1. Materialien

Die beanspruchten Verbindungen können auch unter Verwendung von bekannten Syntheserouten, die dem Fachmann bekannt sind, hergestellt werden. Der Fachmann kann sich hierbei insbesondere an den bekannten Chinolon-Antibiotika orientieren, die die Reste Resten R₁, R₂, R₅ und R₆, so wie hierin definiert, tragen. Beispielsweise könnte so zunächst eine Aminfunktion am Heterozyklus des Chinolongerüsts erzeugt und diese dann mit einer Carbonsäure zu der gewünschten Amidgruppe umgesetzt werden. Als Hilfsmittel bei der Bildung der Amidbindung kommt hierbei Dicyclohexylcarbodiimid (DCC) oder ähnliches in Betracht, wobei die freie Carboxylgruppe am Heterozyklus des Chinolongerüsts in diesem Schritt geschützt werden sollte.

Cip-P2C und Cip-Pro und andere erfindungsgemäße Antibiotika können auch unter Anwendung allgemeinen Fachwissens hergestellt werden. Insbesondere können Cip-P2C und Cip-Pro als Ausgangsmaterialien eingesetzt werden, um weitere erfindungsgemäße Antibiotika herzustellen.

### Bestimmung der minimalen Hemmkonzentrationen in verschiedenen Bakterienstämmen:

Die antibiotische Aktivität wurde durch die Durchführung der Broth-Mikroverdünnungsmethode ermittelt (Cockerill et al., 2012; siehe zitierte Literatur). Es wurden die folgenden Bakterienstämme verwendet: Staphylococcus aureus (ATCC 29213), Enterococcus faecalis (ATCC 29212), Escherichia coli (ATCC 25922) and Pseudomonas aeruginosa (ATCC 27853), sowie S. aureus (ATCC 43300), K. pneumoniae (ATCC 700603), A. baumanii (ATCC 19606).

Bakterienzellen wurden bei einer Zelldichte von 2 - 8 × 105 cfu/ml in 200 µl Mueller-Hinton-Brothmedium verdünnt. Die Ansätze enthielten eine Verdünnungsreihe der Testsubstanzen. Diese inokulierten 96-well Platten wurden für 18 h bei 36°C inkubiert. Die minimale Hemmkonzentration wurde ermittelt durch visuelle Abschätzung der niedrigsten Konzentration, die die Bakterien vollständig gehemmt hatte.

### Zytotoxizitätstest:

Humane embryonale Nierenzellen (HEK293) wurden in 384-well-Platten mit einer Dichte von 6000 Zellen/well in einem finalen Volumen von 50 µl mit der gewünschten Konzentration der Testsubstanz ausgesät. DMEM mit 10% fötalem Rinderserum (FBS) wurde als Wachstumsmedium verwendet. Die Zellen wurden zusammen mit den Testsubstanzen für 20 h bei 37 °C mit 5% CO2 inkubiert.

### Abiotische Hydrolyse:

Ciprofloxacin, Cip-pro oder Cip-P2C wurden in 12 ml phosphat-gepufferter Salzlösung in einer Konzentration von 10 mg/L gelöst. Der pH-Wert wurde auf pH 6, pH 7.4 oder pH 9 eingestellt. Die Ansätze wurden bei 37 °C für pH 6 and 7.4 und bei Raumtemperatur für pH 9 unter Schütteln (250 rpm) für 28 Tage inkubiert. An Tag 0, 1, 7, 14, 21 und 28 wurden Proben (700µL) für die HPLC Analyse entnommen. Der Quotient der Ausgangskonzentration c0 durch die Konzentration am jeweiligen Zeitpunkt (ct) wurde kalkuliert und gegen die Inkubationszeit aufgetragen.

### Wachstumshemmung in E. coli:

Escherichia coli ATCC 23716 wurde von DSMZ GmbH (Braunschweig, Germany) erhalten. Die Testsubstanzen wurden in Wasser verdünnt und auf einer 96-well-Platte zur Bakteriensuspension (in 2x Bakterienmedium (10 g Pepton and 6 g Fleischextrakt in 1 Liter)) gegeben, so dass die Endkonzentration des Mediums 1x war. Die Platte wurde 4h bei 37°C inkubiert. Die Zelldichte wurde photometrisch durch Messung der Absorption bei 600 nm bestimmt. Die Wachstumshemmung in Prozent der unbehandelten Kontrollen gegen die Konzentration der Testsubstanz aufgetragen.

### Hochleistungschromatographie (HPLC) für Ciprofloxacin, Cip-Pro und Cip-P2C:

Das Shimadzu Prominence HPLC System (Duisburg, Germany) wurde für die chromatographischen Untersuchungen der Testsubstanzen verwendet. Eine NUCLEODUR^{®} RP-C18 (CC 125/4 100-5µm C18 ec) Säule und mobile Phasen bestehend aus 0.1 % Ameisensäure in hochreinem Wasser (CH₂O₂: Solution A) and 100 % Acetonitril (CH₃CN: Solution B) wurden verwendet. Die Flußrate wurde auf 0.5 mL min⁻¹ eingestellt. Die Temperatur des Säulenofens wurde auf 25 °C eingestellt und das Injektionsvolumen betrug 10 µL. Ciprofloxacin, Cip-Pro und Cip-P2C eluierten bei den Retentionszeiten [t_{R}] 16.1 min, 16.5 min beziehungsweise 15.2 min. Die Testsubstanzen wurden durch einen UV/Vis Detektor bei 270 nm detektiert. Die Fließgradientenmethode wurde angewendet wie in Tabelle 2 aufgelistet.

**Tabelle 1: Fließgradientenbedingungen zur chromatographischen Analyse der Testsubstanzen**

| **Zeit (min)** | **% Solution B (Acetonitril)** |
|---|---|
| 0.01-2.0 | 1 |
| 5.0 | 5 |
| 7.0 | 10 |
| 10.0 | 15 |
| 13.0 | 25 |
| 15.0 | 30 |
| 17.0 | 35 |
| 20.0 | 40 |
| 22.0 | 50 |
| 25.0 | 60 |
| 27.0 | 65 |
| 29.0 | 20 |
| 30.0-32.0 | 1 |
| 32.01 | Stop |

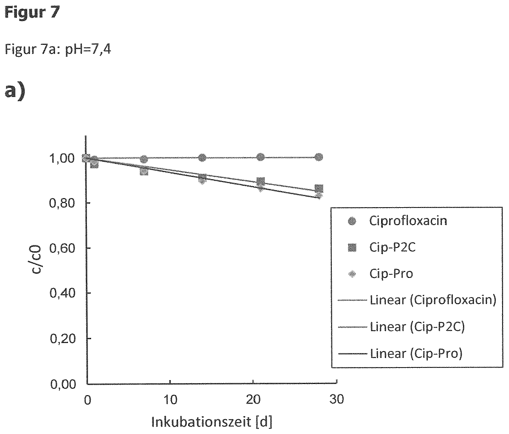

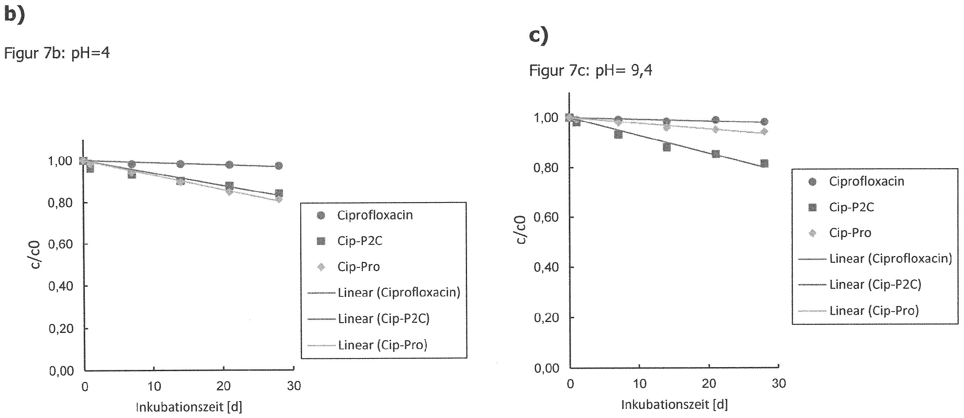

### Abiotische Hydrolyse Ciprofloxacin, Cip-Pro, Cip-P2C (siehe Figuren 7a-c):

pH-Abhängigkeit der abiotischen Hydrolyse bei pH 7,4 (7a), pH 4 (7b) und bei pH 9,4 (7c). 10 mg/ml der Testsubstanzen in phosphat-gepufferter Salzlösung (PBS) wurden bei unterschiedlichen pH-Bedingungen bei Raumtemperatur für 28 Tage inkubiert und durch chromatographische Methoden analysiert. Die Ausgangskonzentration c0 wurde mit der Konzentration am jeweiligen Zeitpunkt (c) in Beziehung gesetzt und gegen die Inkubationszeit aufgetragen.

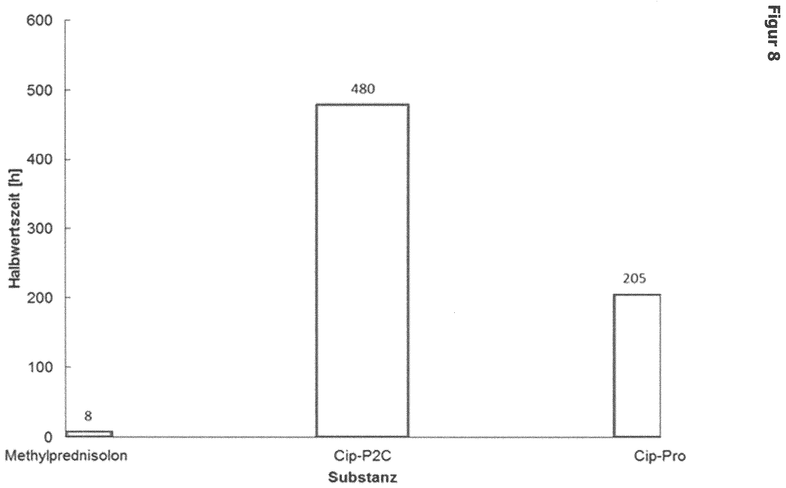

### Enzymatische Hydrolyse durch intestinale Mikrosomen von Ciprofloxacin, Cip-Pro, Cip-P2C (Siehe Figur 8):

Halbswertszeiten der Testsubstanzen bei Inkubation mit humanen intestinalen Mikrosomen. 20 ppm der Testsubstanzen wurden in 1 ml PBS mit 150 µg/ml humanen intestinalen Mikrosomen bei 37 °C unter Schütteln für 24 h inkubiert. Als Positivkontrolle fungierte der Ester Methylprednisolon-hemisuccinat. Die Endkonzentration der Testsubstanzen wurde durch HPLC bestimmt. Aus Ausgangskonzentration und Endkonzentration wurde die Halbwertszeit berechnet.

### Enzymatische Hydrolyse durch intestinale Mikrosomen von Ciprofloxacin, Cip-Pro, Cip-P2C (Figur 8):

Halbswertszeiten der Testsubstanzen bei Inkubation mit humanen intestinalen Mikrosomen. 20 ppm der Testsubstanzen wurden in 1 ml PBS mit 150 µg/ml humanen intestinalen Mikrosomen (Xenotech) bei 37 °C unter Schütteln für 24 h inkubiert. Als Positivkontrolle fungierte der Ester Methylprednisolon-hemisuccinat. Die Endkonzentration der Testsubstanzen wurde durch HPLC bestimmt. Aus Ausgangskonzentration und Endkonzentration wurde die Halbwertszeit berechnet.

In einer Ausführungsform der Erfindung weist das Chinolon-Antibiotikum eine Halbwertszeit von weniger als 50 Tagen oder weniger als 30 Tagen in diesem Test auf.

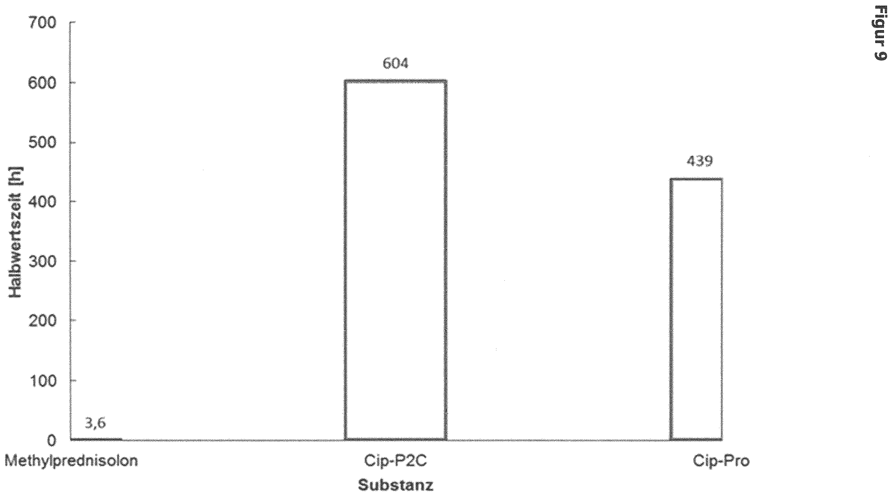

### Enzymatische Hydrolyse (Figur 9):

20 ppm der Testsubstanzen wurden in 1 ml PBS mit 500 µg/ml humanen Lebermikrosomen (Corning) bei 37 °C unter Schütteln für 24 h inkubiert. Die Konzentrationen der Testsubstanzen wurden durch HPLC bestimmt. Aus der Abnahme der Konzentrationen im Verlauf von 24h wurde die Halbwertszeit berechnet.

In einer Ausführungsform der Erfindung weist das Chinolon-Antibiotikum eine Halbwertszeit von weniger als 50 Tagen oder weniger als 30 Tagen in diesem Test auf.

### 2. Synthese

### 2.1 CIP-P2C

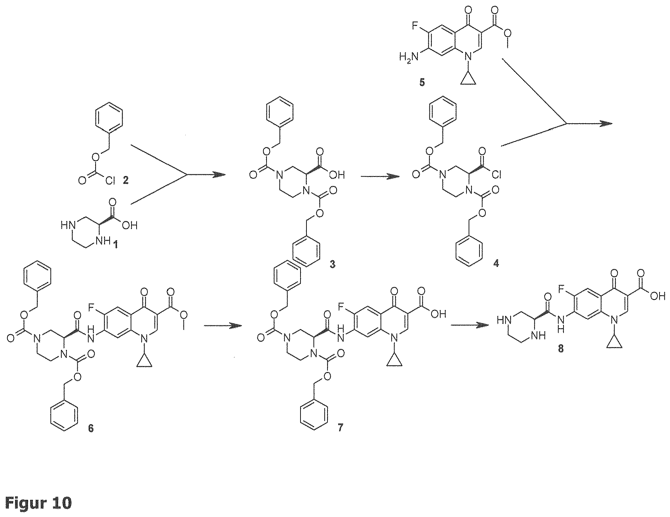

Siehe Figur 10: S-Piperazin-2-carbonsäure (1) reagiert mit Benzylchlorformiat (2) zu 1,4-Cbz-(S)-Piperazin-2-carbonsäure (3), welches nachfolgend mit z.B. Thionylchlorid in Acetonitril (ACN) zum Säurechlorid (4) umgesetzt wird. Dieses reagiert mit (5) zum Dibenzyl(S)-2-((1-cyclopropyl-6-fluoro-3-(methoxycarbonyl)-4-oxo-1,4-dihydroquinolin-7-yl)carbamoyl)piperazin-1,4 dicarboxylat (6), welches mit NaOH zu (7) hydrolysiert werden kann. Abschließend können die Cbz(Benzyloxycarbonyl)-Schutzgruppen beispielsweise mit Pd/C H₂ entfernt werden (8).

Anmerkung: Da (5) nicht käuflich zu erwerben ist, muss dieses über die Gould-Jacobs-Reaktion mit 4-Fluoro-3-nitroanilin als Ausgangsmaterial hergestellt werden. Um zum Amin zu kommen muss die Nitrogruppe folgend mit einem geeigneten Reduktionsmittel umgesetzt werden z.B. mit Pd/C H₂.
▪ 4-Fluoro-3-nitroanilin, Cas. 364-76-1, von Chempur, 1697-25
▪ (S)-Piperazin-2-carbonsäure, Cas. 147650-70-2, von Chempur, 93780-1
▪ Thionyl chlorid, Cas. 7719-09-7, 5 mL von Sigma Aldrich, 230464-5ML
▪ Diethylethoxymethylenmalonat (Cas 87-13-8, von Alfa Aesar, A13776)

### 2.2 CIP-Prolin

Die Synthese von CIP-Prolin kann analog zu CIP-P2C erfolgen. Cbz-L-Prolin (1) wird mit z.B. Thionylchlorid in ACN zum Säurechlorid (2) umgesetzt. Nachfolgend werden (2) und (4) in z.B. ACN mit Triethylamin (TEA) zu (5) umgesetzt. Anschließend wird der Ester mit NaOH zu (6) hydrolysiert und die Cbz-Schutzgruppe mit Pd/C H₂ entfernt (7).

Anmerkung: Da (4) nicht käuflich zu erwerben ist, muss dieses über die Gould-Jacobs-Reaktion mit 4-Fluoro-3-nitroanilin als Ausgangsmaterial hergestellt werden. Um zum Amin zu kommen muss die Nitrogruppe folgend mit einem geeigneten Reduktionsmittel umgesetzt werden z.B. mit Pd/C H₂.
▪ N-Carbobenzoxy-L-prolin (1), Cas. 1148-11-4, von TCI, C0713
▪ Thionylchlorid, Cas. 7719-09-7, von Sigma Aldrich, 230464-5ML
▪ 4-Fluoro-3-nitroanilin, Cas. 364-76-1, von Chempur, 1697-25
▪ Diethyl ethoxymethylenmalonat (Cas 87-13-8, von Alfa Aesar, A13776)

### 2.3 Allgemeine Anmerkungen:

Die vorgestellten Synthesen stellen Standardmethoden in der Synthese von Fluoroquinolonen dar (Gould-Jacobs-Reaktion, SN am Aromaten und Hydrolyse des Esters). Weiterhin ist auch das Schützen des Amins im Fall von CIP-P2C und CIP-Prolin mit der Cbz-Gruppe eine Standardmethode. Weitere generelle Synthesemöglichkeiten für Antibiotika bzw. chemische Reaktionen zum Einfügen bestimmter Gruppen eines Moleküls werden auch in der hierin zitierten Literatur beschrieben.

### Zitierte Literatur:

CN103405435A
CN102030737A
EP1160241A2
EP373531A1
Junza et al., Journal of Agricultural and Food Chemistry, 2014, 62 (8), 2008-2021).
Liu et al., Water Research, 2012, 46(16), 5235-5246
Cockerill, Franklin R. (2012): Methods for dilution antimicrobial susceptibility tests for bacteria that grow aerobically. Approved standard - ninth edition. Wayne, Pa.: CLSI (Clinical and Laboratory Standards Institute, M07-A9 = 32,2).
Wetzstein et al., "Patterns of metabolites produced from the fluoroquinolone enrofloxacin by basidiomycetes indigenous to agricultural sites", Applied Microbioiogy and Biotechnology 2006, Vol71, Nr. 1, Seiten 90-100.
Sauter et al., "Synthesen neuer Chinolon-Chemotherapeutika, 4. Mitt. Pyrido[3,2,1-gh][1,7]phenanthrolin- und Benzo[i,j]chnolizin-carbonsäuren", Scientia Pharmaceutica 1989, 57, 7-20.
WO 2007/106537 A2
JP 57016882 A

## Patentansprüche

1. Chinolon-Antibiotikum der allgemeinen Formel I, II, III oder IV: 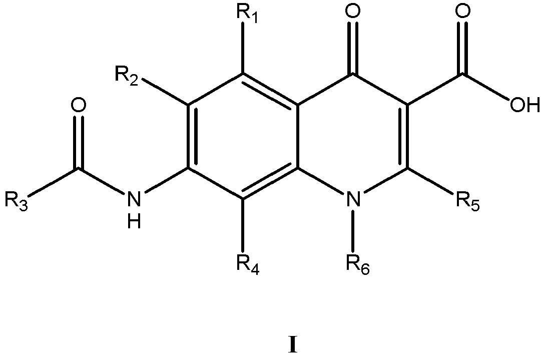 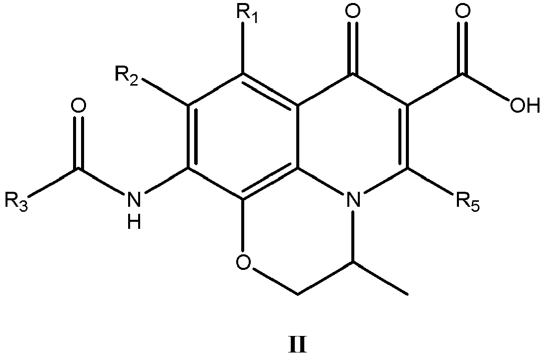 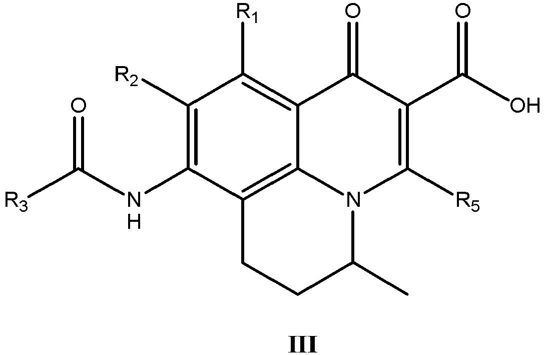 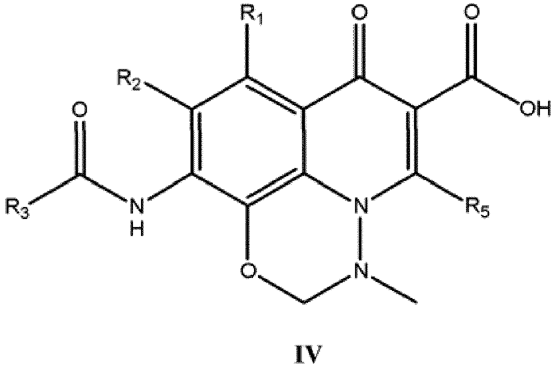 worin
R₁ in Formel I und Formel IV gleich H, Halogen, oder NH₂ist,
R₁ in Formel II und Formel III gleich Halogen, oder NH₂ ist,
R₂ gleich Halogen, oder H ist,
R₃ ist 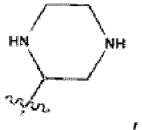
oder der Rest einer Aminosäure nach amidischer Bindung an das Chinolon-Grundgerüst,
wobei die zu R₃ benachbarte Carbonylgruppe aus der Säurefunktion der Aminosäure stammt; wobei die Aminosäure ausgewählt ist aus: 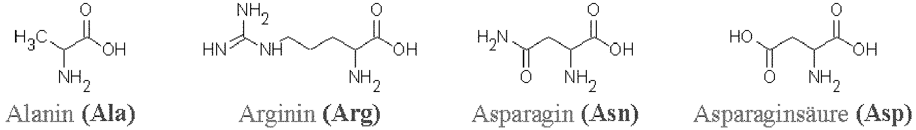 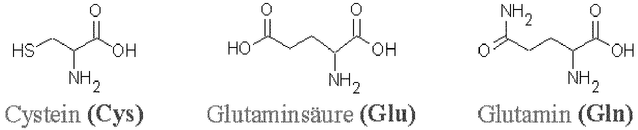 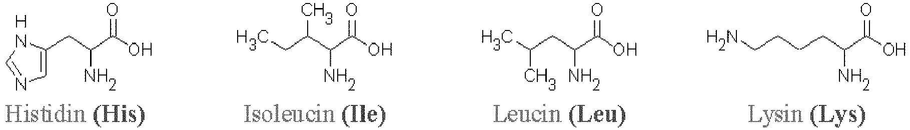 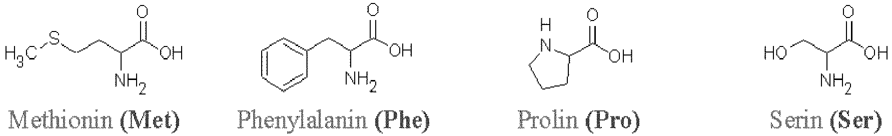 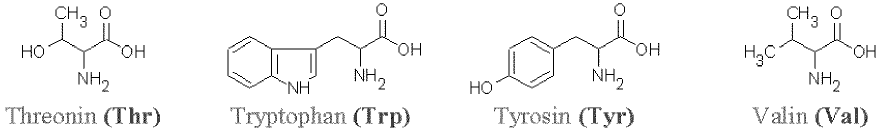
R₄ gleich H, OCH₃, OCHF₂, CN, C₁₋₆ Alkoxy, Halogen, oder NH₂ ist
R₅ gleich H ist
und R₆ ausgewählt ist aus
| Cyclopropyl | Cyclobutyl | Ethyl | Propyl | Methyl |
|---|---|---|---|---|
| 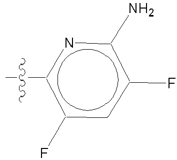 | 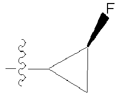 | 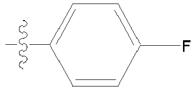 | 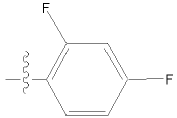 | 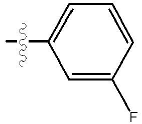 |
| 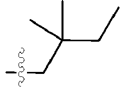 | 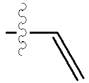 | 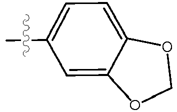 | 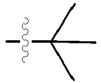 | 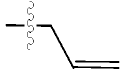 |
| 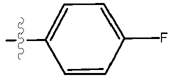 | 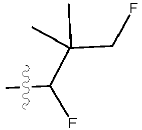 | 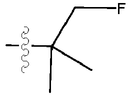 | 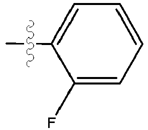 | 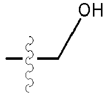 |
| 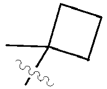 | 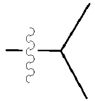 | 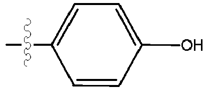 | 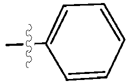 | |
zur Verwendung bei der Behandlung von bakteriellen Infektionen.

2. Das Chinolon-Antibiotikum zur Verwendung nach Anspruch 1 mit der Formel 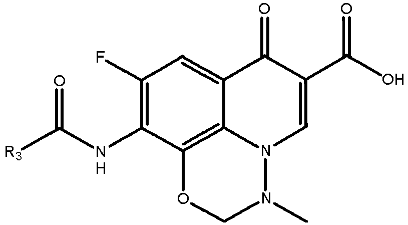 wobei R₃ wie in Anspruch 1 definiert ist.

3. Das Chinolon-Antibiotikum zur Verwendung nach einem der vorangegangenen Ansprüche, wobei
R₁ in Formel I und Formel IV = H, oder F
R₁ in Formel II und Formel III = F
R₂ = H, oder F
R₃ = wie in Anspruch 1 definiert
R₄ = H, OCH₃, OCHF₂, Cl, CN, oder F, und
R₅ = H, ist.

4. Das Chinolon-Antibiotikum zur Verwendung nach einem der vorangegangenen Ansprüche mit der Formel II-a 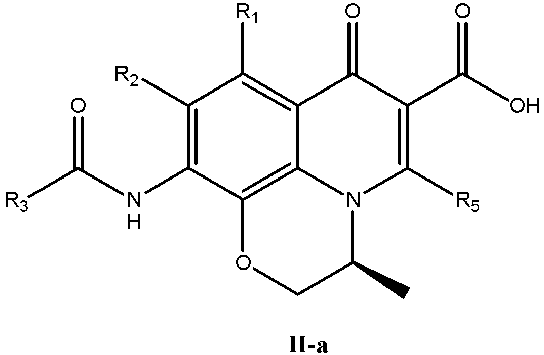 wobei R₁, R₂, R₃ und R₅ wie in Anspruch 1 definiert sind.

5. Chinolon-Antibiotikum zur Verwendung nach einem der vorangegangenen Ansprüche mit der allgemeinen Formel: 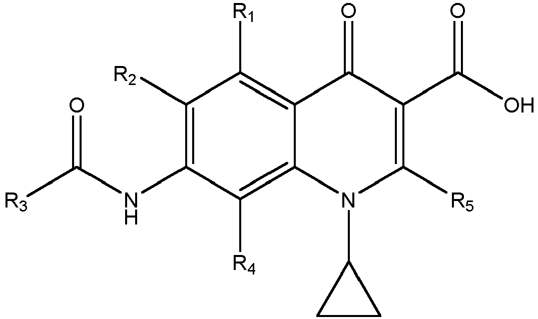 wobei R₁, R₂, R₃, R₄ und R₅ wie in Anspruch 1 definiert sind.

6. Das Chinolon-Antibiotikum zur Verwendung nach einem der vorangegangenen Ansprüche, bei dem die Aminosäure R₃ in L-Konfiguration ist.

7. Das Chinolon-Antibiotikum zur Verwendung nach einem der vorangegangenen Ansprüche, mit der Formel 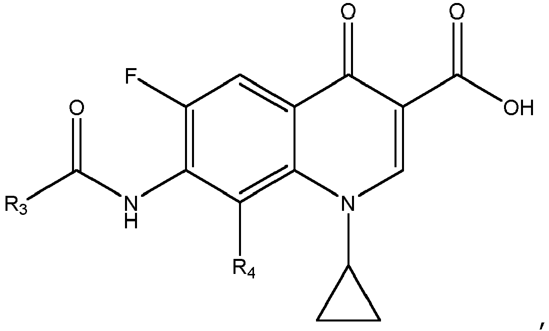 wobei
R₃ eine natürliche Aminosäure nach Anspruch 1 ist, und
R⁴ ein Wasserstoffatom ist.

8. Das Chinolon-Antibiotikum zur Verwendung nach einem der vorangegangenen Ansprüche, wobei die Amidbindung abiotisch oder biotisch abgespalten werden kann.

9. Medikament enthaltend ein Chinolon-Antibiotikum nach einem der vorangegangenen Ansprüche.

## Claims

1. Quinolone antibiotic of the general formula I, II, III or IV: 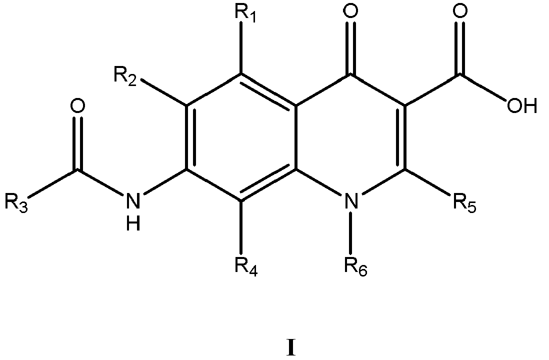 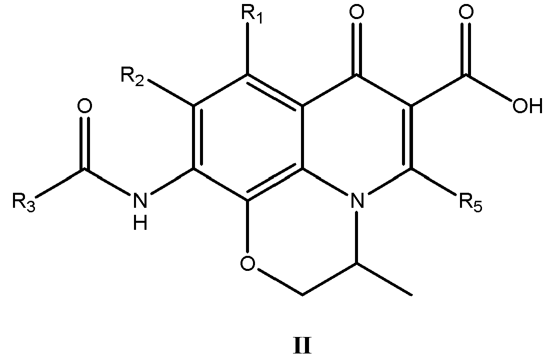 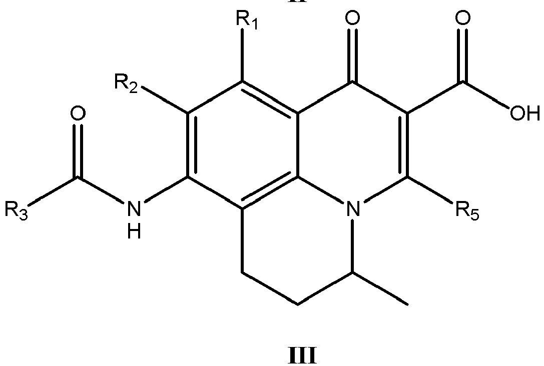 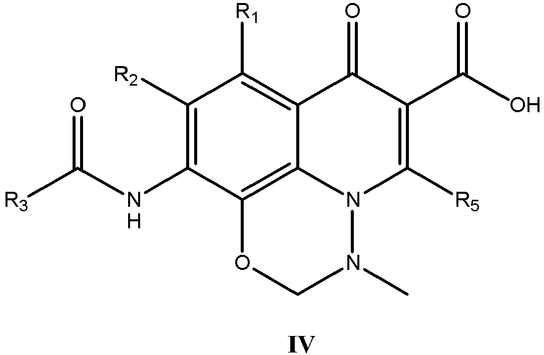 wherein
R₁ in formula I and formula IV is H, halogen, or NH₂,
R₁ in formula II and formula III is halogen, or NH₂,
R₂ is halogen, or H,
R₃ is 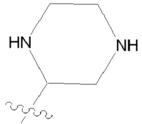
or the residue of an amino acid after amidic bonding to the quinolone basic skeleton,
wherein the carbonyl group adjacent to R₃ is derived from the acid function of the amino acid; wherein the amino acid is selected from: 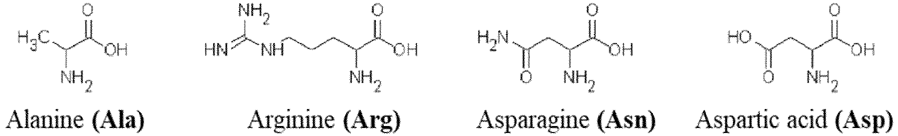 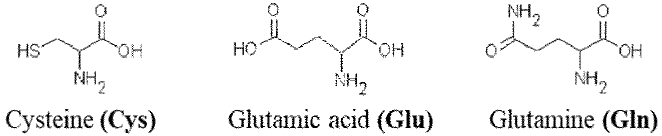 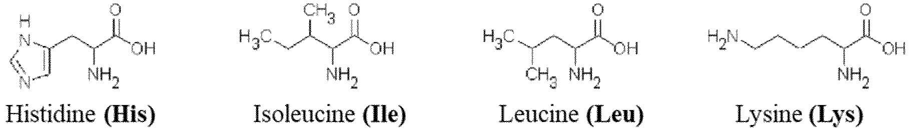 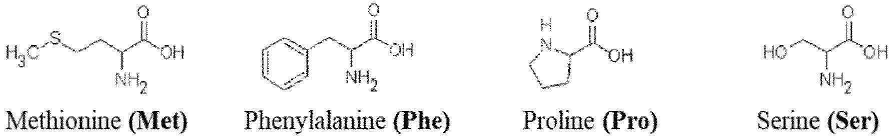 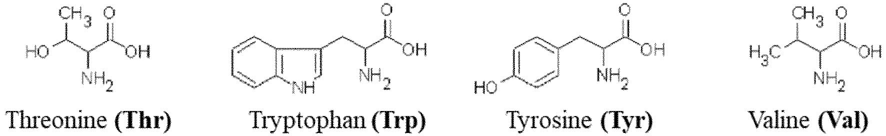
R₄ is H, OCH₃, OCHF₂, CN, C₁₋₆ alkoxy, halogen, or NH₂
R₅ is H
and R₆ is selected from
| Cyclopropyl | Cyclobutyl | Ethyl | Propyl | Methyl |
|---|---|---|---|---|
| 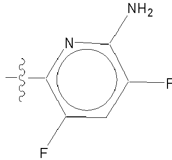 | 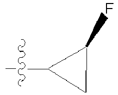 | 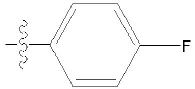 | 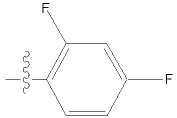 | 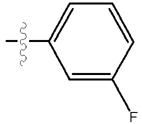 |
| 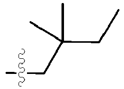 | 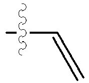 | 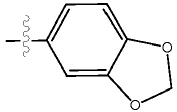 | 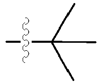 | 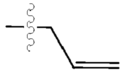 |
| 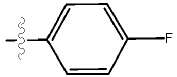 | 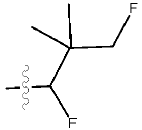 | 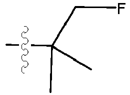 | 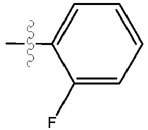 | 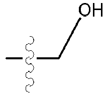 |
| 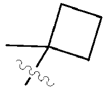 | 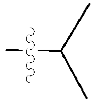 | 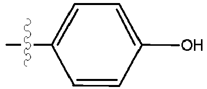 | 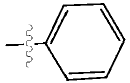 | |
for use in the treatment of bacterial infections.

2. The quinolone antibiotic for use according to claim 1 of the formula 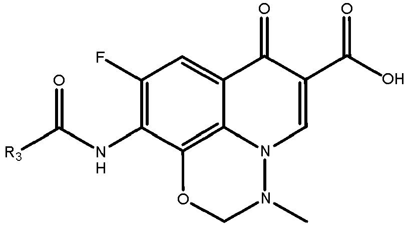 wherein R₃ is as defined in claim 1.

3. The quinolone antibiotic for use according to any one of the preceding claims, wherein
R₁ in formula I and formula IV = H, or F
R₁ in formula II and formula III = F
R₂ = H, or F
R₃ = as defined in claim 1
R₄ = H, OCH₃, OCHF₂, Cl, CN, or F, and
R₅ = H.

4. The quinolone antibiotic for use according to any one of the preceding claims of the formula II-a 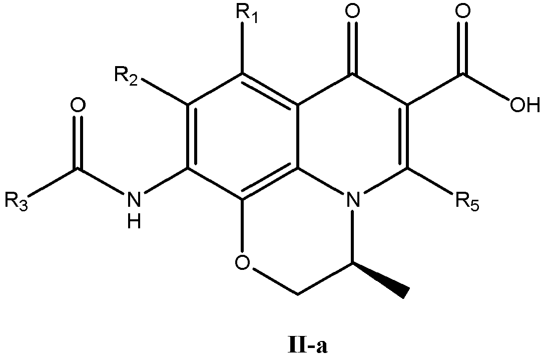 wherein R₁, R₂, R₃ and R₅ are as defined in claim 1.

5. Quinolone antibiotic for use according to any one of the preceding claims of the general formula: 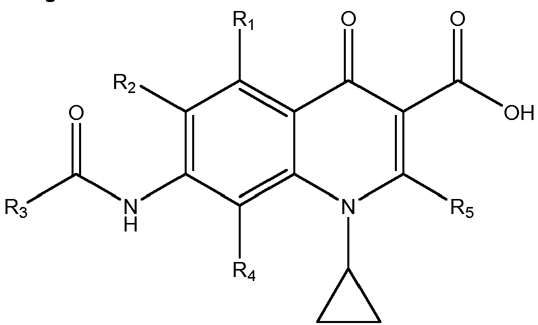 wherein R₁, R₂, R₃, R₄ and R₅ are as defined in claim 1.

6. The quinolone antibiotic for use according to any one of the preceding claims, wherein the amino acid R₃ is in L-configuration.

7. The quinolone antibiotic for use according to any one of the preceding claims, of the formula 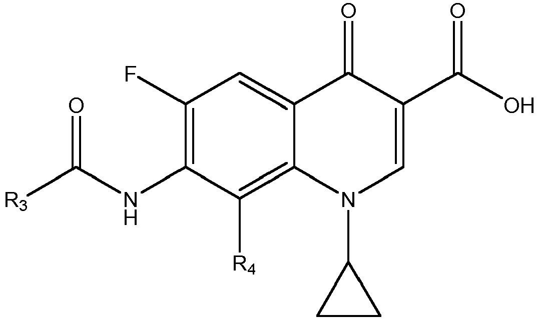 wherein R₃ is a natural amino acid according to claim 1, and
R₄ is a hydrogen atom.

8. The quinolone antibiotic for use according to any one of the preceding claims, wherein the amide bond can be cleaved abiotically or biotically.

9. Medicament containing a quinolone antibiotic according to any one of the preceding claims.

## Revendications

1. Antibiotique de type quinolone de formule générale I, II, III ou IV : 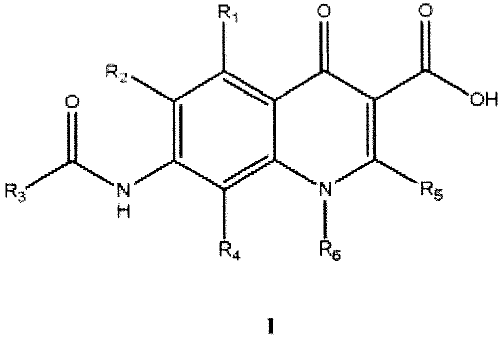 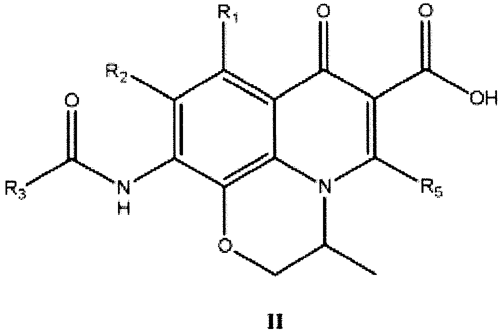 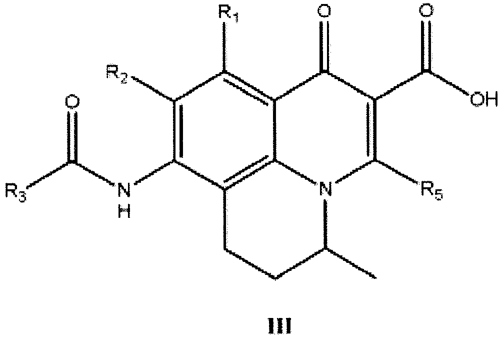 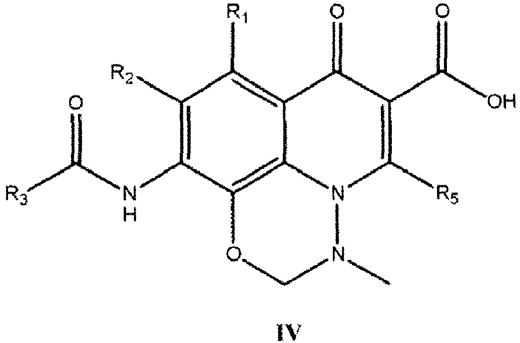 dans laquelle
R₁ dans la formule I et la formule IV est H, un halogène ou NH₂,
R₁ dans la formule II et la formule III est un halogène ou NH₂,
R₂ est un halogène ou H,
R₃ est 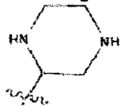
ou le reste d'un acide aminé après liaison amidique au squelette de la quinolone, le groupe carbonyle adjacent à R₃ provenant de la fonction acide de l'acide aminé ; l'acide aminé étant choisi parmi : 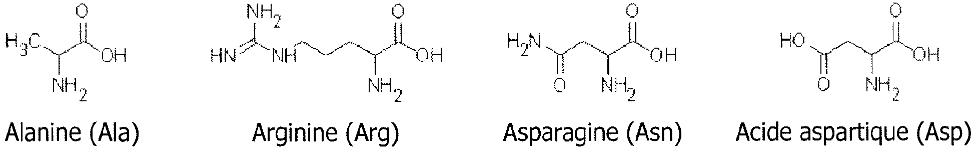 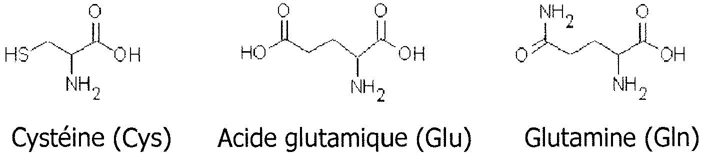 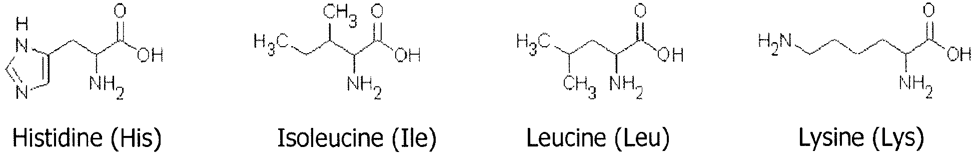 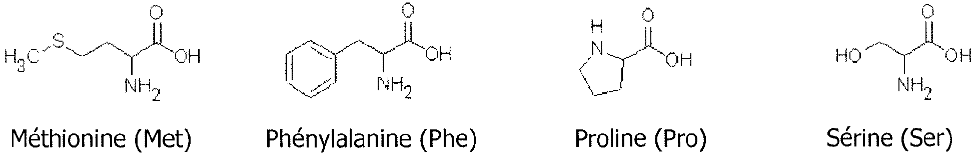 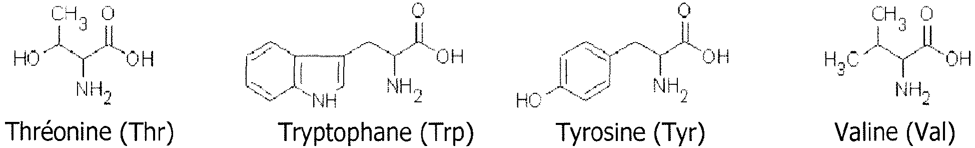
R₄ est H, OCH₃, OCHF₂, CN, un alcoxy en C₁₋₆, un halogène ou NH₂
R₅ est H
et R₆ est choisi parmi :
| Cyclopropyle | Cyclobutyle | Éthyle | Propyle | Méthyle |
|---|---|---|---|---|
| 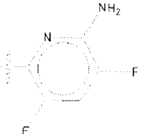 | 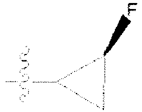 | 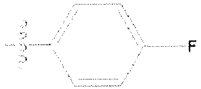 | 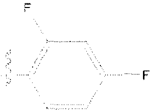 | 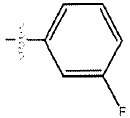 |
| 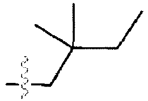 | 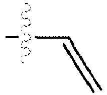 | 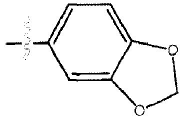 | 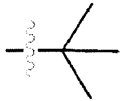 | 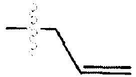 |
| 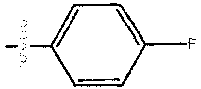 | 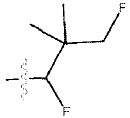 | 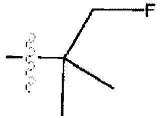 | 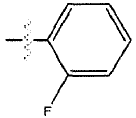 | 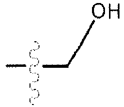 |
| 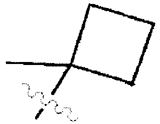 | 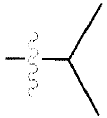 | 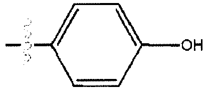 | 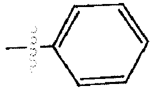 | |
destiné à être utilisé dans le traitement d'infections bactériennes.

2. Antibiotique de type quinolone destiné à être utilisé selon la revendication 1, ayant la formule 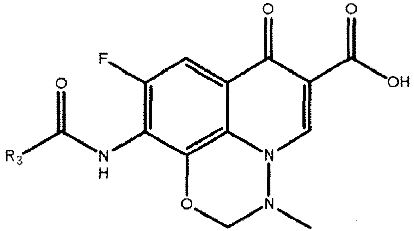 dans laquelle R₃ est tel que défini dans la revendication 1.

3. Antibiotique de type quinolone destiné à être utilisé selon l'une des revendications précédentes, dans lequel
R₁ dans la formule I et la formule IV = H ou F
R₁ dans la formule II et la formule III = F
R₂ = H ou F
R₃ = tel que défini dans la revendication 1
R₄ = H, OCH₃, OCHF₂, CI, CN ou F, et
R₅ = H.

4. Antibiotique de type quinolone destiné à être utilisé selon l'une des revendications précédentes, ayant la formule II-a 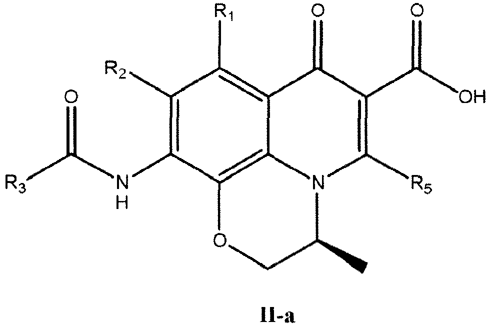 dans laquelle R₁, R₂, R₃ et R₅ sont tels que définis dans la revendication 1.

5. Antibiotique de type quinolone destiné à être utilisé selon l'une des revendications précédentes, ayant la formule générale : 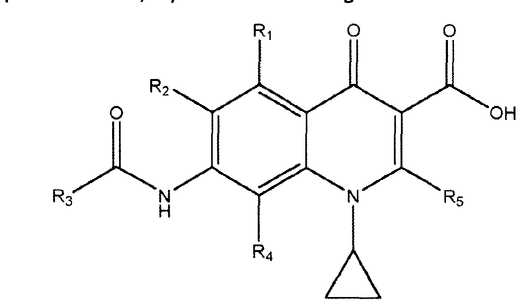 dans laquelle R₁, R₂, R₃, R₄ et R₅ sont tels que définis dans la revendication 1.

6. Antibiotique de type quinolone destiné à être utilisé selon l'une des revendications précédentes, dans lequel l'acide aminé R₃ est en configuration L.

7. Antibiotique de type quinolone destiné à être utilisé selon l'une des revendications précédentes, ayant la formule 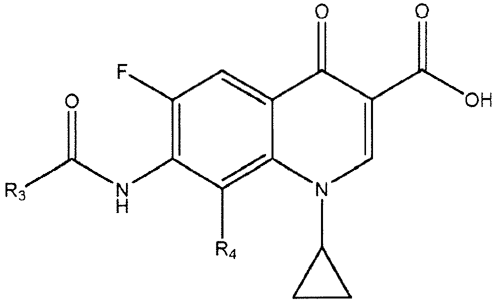 dans laquelle
R₃ est un acide aminé naturel selon la revendication 1, et R₄ est un atome d'hydrogène.

8. Antibiotique de type quinolone destiné à être utilisé selon l'une des revendications précédentes, dans lequel la liaison amide peut être clivée de manière abiotique ou biotique.

9. Médicament contenant un antibiotique de type quinolone selon l'une des revendications précédentes.
